# EUROPÄISCHE PATENTSCHRIFT

(11) **EP 2 609 889 B1**
(45) Veröffentlichungstag und Bekanntmachung des Hinweises auf die Patenterteilung: **17.09.2014**
(21) Anmeldenummer: 12196070.2
(22) Anmeldetag: 07.12.2012
(51) Int. Cl.: A61B 19/02, A61B 19/08

(54) **Auffangschale**
Collection tray
Plateau de collecteur

(30) Priorität: 29.12.2011 DE 202011052539 U
(43) Veröffentlichungstag der Anmeldung: 03.07.2013
(73) Patentinhaber: Winkelmann, Wolfgang, 72076 Tübingen (DE)
(72) Erfinder: Winkelmann, Wolfgang, 72076 Tübingen (DE)
(74) Vertreter: Wohlfahrt, Jan Günther

(56) Entgegenhaltungen:
- DE-U1-202006 004 494
- US-A- 5 220 699
- US-A1- 2010 174 415
- "Hair Wash / Rinse Tray", , 8. Juni 2009 (2009-06-08), XP055055955, Gefunden im Internet: URL:http://www.ezcaremedical.com/store/ind ex.php?main_page=product_info&cPath=546_10 0_136&products_id=516 [gefunden am 2013-03-11]
- "Hair washing tray comfort cape", , 27. September 2010 (2010-09-27), XP055055957, Gefunden im Internet: URL:http://web.archive.org/web/20100927233 211/http://www.completecareshop.co.uk/disa bility-elderly-aid/1110/hair_washing_tray_ comfort_cape.html [gefunden am 2013-03-11]

## Beschreibung

Die Erfindung betrifft eine Auffangschale für Anwendungen bei einer medizinischen und/oder kosmetischen Behandlung eines Patienten, in welcher insbesondere medizinische Instrumente und Hilfsmittel sowie gegebenenfalls auch Zahnersatz zumindest während des Zeitraums der Behandlung in Reichweite einer behandelnden Person, insbesondere eines Arztes, aufgefangen werden können, wobei die Auffangschale einen als Ablagefläche gestalteten Schalenboden umfasst, der von einem umlaufenden Rand umgeben ist.

Derartige Vorrichtungen sind im medizinischen Bereich als Hilfsmittel während der Behandlung von Patienten, insbesondere bei Eingriffen und Operationen seit langem bekannt.

Die bei weitem häufigste Verwendung bei der zahnärztlichen Behandlung bzw. auch allgemein bei der Patienten-Behandlung im Kopf / Hals-Bereich finden sogenannte "Patienten-Umhänge", die auf den Oberkörper Hals-Brust-Bauch-Bereich aufgelegt bzw. dort befestigt werden. Diese Umhänge (-aus verschiedensten Materialien wie Papier, Polyethylen etc., mit oder ohne Zellstoffauflage zum Aufsaugen von Flüssigkeiten) werden entweder mit Bändern oder Kettchen bzw. direkt am Hals des Patienten festgebunden, und dienen vor allem dem Schutz des Patienten bzw. seiner Kleidung vor Verschmutzung oder Beschädigung durch Substanzen und Chemikalien, die bei der Behandlung -eigentlich intraoral- erforderlich sind.

Spezielle Ausführungen dieser Umhänge haben außerdem am unteren Rand zusätzlich eine sog. "Auffangtasche" zum Auffangen von Flüssigkeiten oder herunter laufenden Substanzen ähnlich den bei Kleinkindern gebräuchlichen Umhängen ("Schlabberlätzchen" mit unterer Auffangkante). Eine solche -nur am unteren Rand im Bauchbereich angebrachte- Auffangkante macht jedoch nur Sinn, wenn der Patient aufrecht, oder halb-aufrecht sitzt. Selbst dann ist aber das Abgleiten von Kleinteilen, Kronen, Brücken, Implantaten, Implantatschrauben u.ä. über die Schulter des Patienten, und das anschließende Herunterfallen auf den Boden nicht zu verhindern. Dieser Mangel wird noch gravierender, wenn der Patient im Liegen bzw. in annähernd waagerechter Position behandelt wird, wie es heute bei den meisten Behandlungssituationen und Behandlungskonzepten im zahnärztlichen Bereich der Fall ist. Eine alleinige untere "Auffangkante" wäre dabei nutzlos.

Unter dem Namen "'Bodytray" findet man im Internet eine Art Ablage/ Tablett, welches für die Dauer der Behandlung auf den Brust/Bauchbereich des waagerecht liegenden Patienten aufgesetzt wird, und ausschließlich als Ablagefläche ("Bauch-Tablett") für Instrumente o.ä. dient. Dieses Produkt ist wohl in erster Linie gedacht als Ersatz bei fehlendem fest installierten sogenanntem "Schwebetisch". Auch dieses "Body"-Tray verhindert nicht ein Abgleiten, bzw. Herunterfallen von Kleinteilen beim Transport vom Mund auf dieses Tray oder umgekehrt, da dabei der Abstand zwischen Tray und Mund des Patienten genauso groß ist und mithin die Kleinteile nicht am Herunterfallen gehindert werden.

Im gesamten medizinischen und pflegerischen Bereich ist seit langer Zeit die sogenannte "Nierenschale" wohlbekannt, die ebenfalls aus verschiedenen Materialien und in einer Standard-Form und Standard-Größe angeboten wird. Auch diese "Auffangschale in Nierenform" erfüllt nicht die oben geschilderten Anforderungen insbesondere im zahnärztlichen Bereich. Dazu müsste die "Nierenschale" nämlich eigentlich permanent mit den Händen festgehalten werden. Sie ist sowohl von der Form als auch von ihren Abmessungen zu klein, zu kurz, anatomisch dem menschlichen Oberkörper nicht angepasst und für den hier angestrebten Einsatzzweck des selbsttätigen sicheren Auffangens von herunterfallenden Kleinteilen weder geeignet noch vergleichbar.

US Patentanmeldung 2010/0174415 A1 offenbart den Gegenstand der Präambel von Anspruch 1.

Aufgabe der vorliegenden Erfindung ist es demgegenüber, eine gattungsgemäße Auffangschale der eingangs beschriebenen Art mit möglichst simplen und preisgünstigen technischen Mitteln dahin gehend zu verbessern, dass sie gleichzeitig folgende Funktionen erfüllt, nämlich
1. selbsttätiges Auffangen von herunterfallenden Kleinteilen
2. Schutz des Oberkörpers bzw. der Patientenkleidung vor Verschmutzung oder Beschädigung
3. Ablagemöglichkeit für Behandlungsinstrumente
4. ergonomisch günstige Handhabung.

Erfindungsgemäß wird diese Aufgabe auf ebenso überraschend einfache wie wirkungsvolle Art und Weise vollständig dadurch gelöst, dass die Auffangschale eine Aufnahmeaussparung aufweist, welche sich von einem Teilbereich des umlaufenden Randes in Richtung eines Zentralbereiches der Schale erstreckt und derart geformt ist, dass sie den Hals eines auf dem Rücken liegenden Patienten mit geringem Abstand zum Patienten beidseitig etwa bis in den Bereich der Ohren umgibt, dass der an die Aufnahmeaussparung anschließende Zentralbereich der Auffangschale sich im aufgelegten Zustand der Schale zumindest über einen Teil der Brust des Patienten erstreckt, dass die Aufnahmeaussparung von zwei Auslegern begrenzt ist, welche sich links und rechts des liegenden Patienten im aufgelegten Zustand der Schale zu beiden Seiten seines Halses erstrecken, wobei die Ausleger in einem Bereich zwischen der Aufnahmeaussparung und dem vom Patienten abgewandten Rand der Schale eine Ablagefläche bilden, dass der vom Schalenboden und dem umlaufenden Rand gebildete muldenartige Ablagebereich jeweils an den beiden Auslegern, vorzugsweise in dem im aufgelegten Zustand der Schale den Schultern des Patienten benachbarten Bereich, seine größte Tiefe aufweist, und dass der vom Schalenboden und dem umlaufenden Rand gebildete Ablagebereich in dem im aufgelegten Zustand der Schale auf der Brust des Patienten aufliegenden Bereich eine gegenüber dem umgebenden Schalenboden tiefere Mulde bildet.

Beim Gebrauch liegt die erfindungsgemäße Auffangschale auf dem Oberkörper des liegenden Patienten im Brustbereich auf ohne dabei abzurutschen. Dadurch können bei der Behandlung des Patienten im Kopfbereich herunterfallende Kleinteile, etwa Zahnersatz, Plomben, Kronen etc., sicher und unbeschädigt in der Schale aufgefangen sowie der Patient vor Verschmutzung geschützt werden. Außerdem bietet die Schale dem behandelnden Arzt eine bequeme und saubere Ablage bzw. Bereitstellungsfläche für Instrumente und Behandlungsmaterial, dass die Aufnahmeaussparung von zwei Auslegern begrenzt ist, welche sich links und rechts des liegenden Patienten im aufgelegten Zustand der Schale zu beiden Seiten seines Halses erstrecken, wobei die Ausleger in einem Bereich zwischen der Aufnahmeaussparung und dem vom Patienten abgewandten Rand der Schale eine Ablagefläche bilden. In der tieferen Mulde am Schalenboden können insbesondere Behandlungsmaterial und ärztliche Instrumente bereitgestellt bzw. abgelegt werden.

Ästhetisch hochwertig und mnemotechnisch günstig für die unmittelbare Erkennung des beabsichtigten Verwendungszweckes im zahnärztlichen Bereich sind Ausführungsformen der Erfindung, bei denen die beiden Ausleger, die Aufnahmeaussparung und der Zentralbereich der Auffangschale in einer Draufsicht auf die Schalenfläche die Form eines stilisierten Backenzahnes aufweisen, dessen Zahnwurzeln durch die beiden Ausleger gebildet wird.

Besonders günstig in der Handhabung und zuträglich für eine erhöhte Bequemlichkeit des Patienten während der Behandlung sind Ausführungsformen, bei welchen die Aufnahmeaussparung an ihrem während der Behandlung dem Patienten zuwandten Rand eine Polsterung aufweist.

Bei herstellungstechnisch vorteilhaften und Materialsparenden Weiterbildungen dieser Ausführungsformen umfasst die Polsterung nur denjenigen Teil des Randbereiches der Aufnahmeaussparung, welcher während der Behandlung dem Hals des Patienten nahekommt.

Andere bevorzugte Weiterbildungen zeichnen sich dadurch aus, dass die Polsterung so gestaltet ist, dass sie während der Behandlung das Kinn des Patienten unterstützt und/oder anhebt.

In der Praxis wird die Polsterung aus Weichmaterial, insbesondere Zellstoff oder Weich-Kunststoff aufgebaut sein, was auch das Gewicht der auf der Brust des Patienten aufliegenden Auffangschale gering hält.

Besonders günstig sind Weiterbildungen, bei welchen das Material der Polsterung so gewählt ist, dass die Polsterung während der Behandlung des Patienten den Raum zwischen Patient und Auffangschale flüssigkeitsdicht abdichtet. Insbesondere im Bereich zahnärztlicher Behandlungen ist dies von großem Vorteil für das Wohlbefinden des behandelten Patienten und macht andere aufwändige "Abdichtmaßnahmen" überflüssig.

In der Handhabung besonders geschickt sind Ausführungsformen der erfindungsgemäßen Auffangschale, die sich dadurch auszeichnen, dass am umlaufenden Rand im Bereich der Aufnahmeaussparung patientenseitig zumindest abschnittsweise Klettband angeordnet ist, mit dessen Hilfe die Schale leicht an der Kleidung des Patienten befestigt und nach der Behandlung wieder abgenommen werden kann.

Bei einer weiteren bevorzugten Ausführungsform der erfindungsgemäßen Auffangschale ist der umlaufende Rand gebördelt. Dadurch kann ein Herausfallen von in die Schale gefallenen oder gelegten Objekten sicher verhindert werden.

Eine besondere Absicherung gegen Herausfallen wird bei Weiterbildungen dieser Ausführungsformen erreicht, bei welchen die Bördelung des umlaufenden Randes an ihrem oberen Ende über den Schalenboden überhängend gestaltet ist.

Die Bördelung muss aber nicht notwendig vollständig umlaufend gestaltet sein. So können auch Weiterbildungen Vorteile aufweisen, bei denen in dem im aufgelegten Zustand der Schale auf der Brust des Patienten aufliegenden Bereich keine Bördelung des umlaufenden Randes vorgesehen ist.

Bei einer Klasse von Ausführungsformen der erfindungsgemäßen Auffangschale für den medizinischen Bedarf besteht die Schale aus sterilisierbarem Material, insbesondere aus Metall, vorzugsweise aus rostfreiem Edelstahl. Diese Ausführungsformen sind - nahezu beliebig oft - wieder verwendbar, allerdings sowohl in der Herstellung als auch in der Handhabung aufwändig und teuer.

Eine alternative - besonders preisgünstige - Klasse von Ausführungsformen zeichnet sich dadurch aus, dass die Schale zur einmaligen Verwendung und anschließenden Entsorgung vorgesehen ist.

Hier kann es vorteilhaft sein, wenn die Schale aus - insbesondere sterilisierbarem - Kunststoff besteht.

Eine günstige Variante dieser Weiterbildungen, zeichnet sich dadurch aus, dass die Schale aus einem zumindest über einen Zeitraum von mehr als 30min feuchtigkeitsdichten Material, vorzugsweise aus Presspappe besteht.

Besonders bevorzugt schließlich sind sehr preisgünstige und geometrisch variable Ausführungsformen der erfindungsgemäßen Auffangschale, bei denen der umlaufende Rand aufblasbar gestaltet ist.

Weitere Merkmale und Vorteile der Erfindung ergeben sich aus der nachfolgenden detaillierten Beschreibung eines Ausführungsbeispiels der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung ist ein Ausführungsbeispiel der Erfindung dargestellt, welches in der nachfolgenden Beschreibung näher erläutert wird.

Es zeigen:

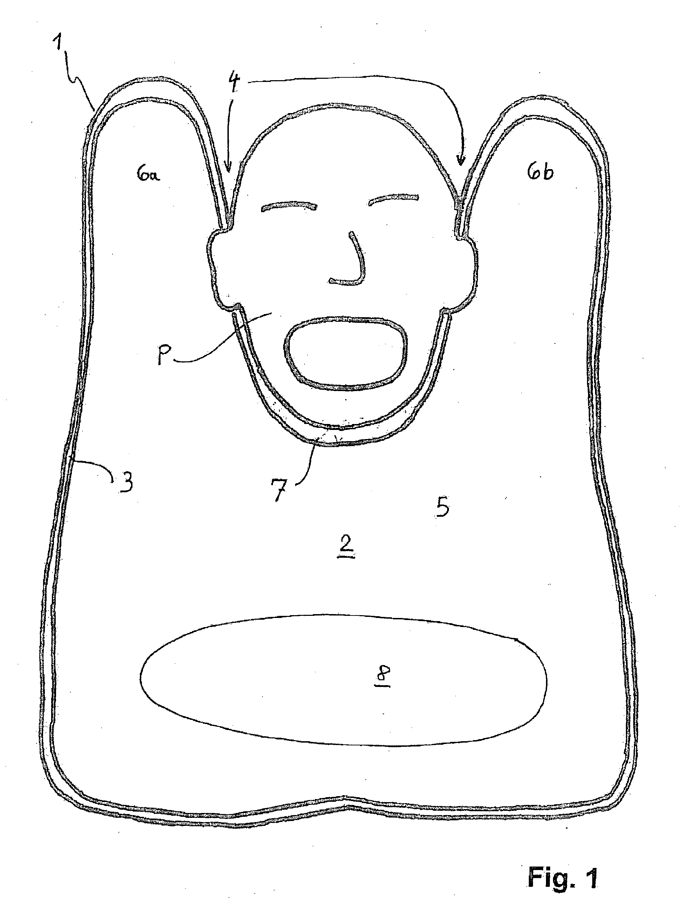

- Fig. 1: einen schematischen Blick von oben auf den Kopf-Brust-Bereich eines Patienten mit angelegter erfindungsgemäßen Auffangschale;

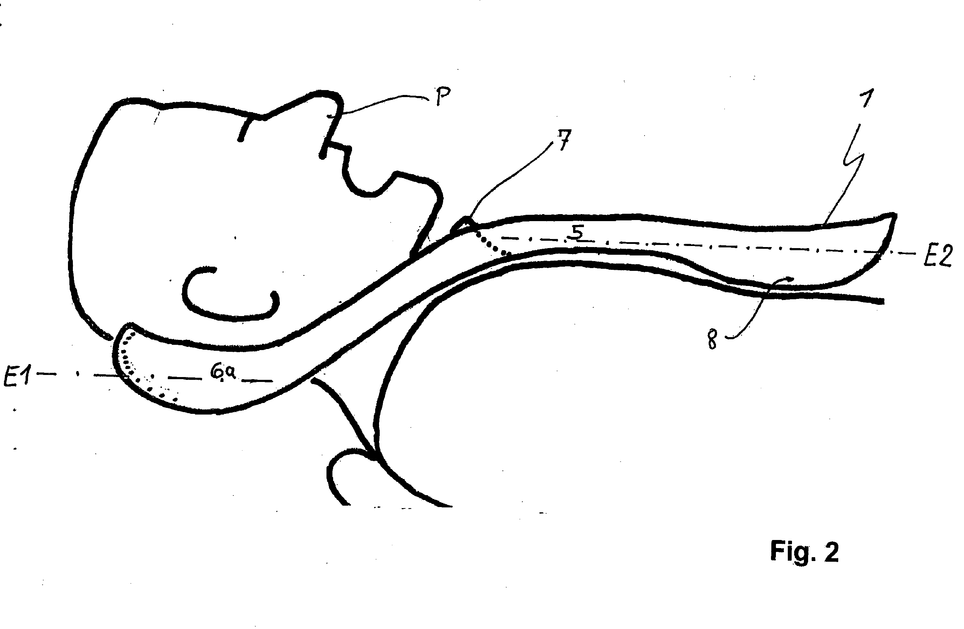

- Fig. 2: die Ausführungsform nach Fig.1 schematisch von der Seite.

Die in den Figuren der Zeichnung schematisch dargestellte Ausführungsform einer erfindungsgemäßen **Auffangschale 1** für Anwendungen bei einer medizinischen und/oder kosmetischen Behandlung eines **Patienten P,** in welcher insbesondere medizinische Instrumente und Hilfsmittel sowie gegebenenfalls auch Implantate oder Gewebe- und Knochenpartikel zumindest während des Zeitraums der Behandlung in Reichweite einer behandelnden Person, insbesondere eines Arztes, aufgefangen werden können, wobei die Auffangschale 1 einen als Ablagefläche gestalteten **Schalenboden 2** umfasst, der von einem **umlaufenden Rand 3** umgeben ist, zeichnet sich dadurch aus, dass die Auffangschale 1 eine **Aufnahmeaussparung 4** aufweist, welche sich von einem Teilbereich des umlaufenden Randes 3 in Richtung eines **Zentralbereiches 5** der Schale 1 erstreckt und derart geformt ist, dass sie den Hals eines auf dem Rücken liegenden Patienten P mit geringem Abstand zum Patienten P beidseitig etwa bis in den Bereich der Ohren umgibt, und dass der an die Aufnahmeaussparung 4 anschließende Zentralbereich 5 der Auffangschale 1 sich im aufgelegten Zustand der Schale 1 zumindest über einen Teil der Brust des Patienten P erstreckt.

Die Aufnahmeaussparung 4 ist von zwei **Auslegern 6a, 6b** begrenzt, die sich links und rechts des liegenden Patienten P im aufgelegten Zustand der Schale 1 zu beiden Seiten seines Halses erstrecken, wobei die Ausleger 6a, 6b in einem Bereich zwischen der Aufnahmeaussparung 4 und dem vom Patienten P abgewandten Rand der Schale 1 eine Ablagefläche bilden.

Die Aufnahmeaussparung 4 weist an ihrem während der Behandlung dem Patienten P zuwandten Rand eine **Polsterung 7** auf, die nur den Randbereich der Aufnahmeaussparung 4 umfasst, welcher während der Behandlung dem Hals des Patienten P nahekommt. Die Polsterung 7 ist aus Weichmaterial, insbesondere Zellstoff oder Weich-Kunststoff aufgebaut und so gestaltet, dass sie während der Behandlung das Kinn des Patienten P unterstützt und/oder anhebt. Das Material der Polsterung 7 ist vorteilhafterweise so gewählt, dass die Polsterung 7 während der Behandlung des Patienten P den Raum zwischen Patient P und Auffangschale 1 flüssigkeitsdicht abdichtet.

Die erfindungsgemäße Auffangschale 1 kann dauerhaft aus Metall, etwa aus rostfreiem Edelstahl, aus Kunststoff oder vorzugsweise aus einem recycelbaren Material bestehen und nur zur einmaligen Verwendung und anschließenden Entsorgung vorgesehen sein. Günstig ist hier die Verwendung eines zumindest über einen Zeitraum von mehr als 30min feuchtigkeitsdichten Materials, beispielsweise Presspappe.

Der vom Schalenboden 2 und dem umlaufenden Rand 3 gebildete Ablagebereich in dem im aufgelegten Zustand der Schale 1 auf der Brust des Patienten P aufliegenden Bereich bildet bei der in der Zeichnung dargestellten Ausführungsform eine gegenüber dem umgebenden Schalenboden 2 vertiefte **Mulde 8.**

Der umlaufende Rand 3 kann gebördelt sein, wobei die Bördelung an ihrem oberen Ende über den Schalenboden 2 überhängend gestaltet sein kann. In dem im aufgelegten Zustand der Schale 1 auf der Brust des Patienten P aufliegenden Bereich kann - bei in der Zeichnung nicht dargestellten Ausführungsformen - die Bördelung des umlaufenden Randes 3 fehlen. Außerdem kann der vom Schalenboden 2 und dem umlaufenden Rand 3 gebildete muldenartige Ablagebereich jeweils an den beiden Auslegern 6a, 6b, vorzugsweise in dem im aufgelegten Zustand der Schale 1 den Schultern des Patienten P benachbarten Bereich, seine größte Tiefe aufweisen. Auch kann besonders variablen Ausführungsformen der umlaufende Rand 3 aufblasbar gestaltet ist.

Bevorzugte Abmaße der erfindungsgemäßen Auffangschale 1 liegen in Längsrichtung, also in Achsrichtung des Patienten P zwischen etwa 40cm und 50cm, wobei sich die Aufnahmeaussparung 4 etwa 10cm bis 20cm in dieser Richtung erstreckt und quer dazu eine mittlere Ausdehnung von etwa 15cm bis 20cm aufweist. Im Halsbereich des Patienten P erstreckt sich die Auffangschale 1 in Querrichtung etwa zwischen 30cm und 35cm, während sie im Brustbereich des Patienten P eine Querausdehnung zwischen etwa 35cm und 45cm aufweisen wird. Es kann auch ein Sortiment von unterschiedlichen Schalengrößen zur optimalen Anpassung an verschieden große Patienten vorgehalten werden.

## Patentansprüche

1. Auffangschale (1) für Anwendungen bei einer medizinischen und/oder kosmetischen Behandlung eines Patienten (P), in welcher insbesondere medizinische Instrumente und Hilfsmittel sowie gegebenenfalls auch Zahnersatz zumindest während des Zeitraums der Behandlung in Reichweite einer behandelnden Person, insbesondere eines Arztes, aufgefangen werden können, wobei die Auffangschale (1) einen als Ablagefläche gestalteten Schalenboden (2) umfasst, der von einem umlaufenden Rand (3) umgeben ist, wobei die Auffangschale (1) eine Aufnahmeaussparung (4) aufweist, welche sich von einem Teilbereich des umlaufenden Randes (3) in Richtung eines Zentralbereiches (5) der Schale (1) erstreckt und derart geformt ist, dass sie den Hals eines auf dem Rücken liegenden Patienten mit geringem Abstand zum Patienten beidseitig etwa bis in den Bereich der Ohren umgibt,
wobei die Aufnahmeaussparung (4) von zwei Auslegern (6a, 6b) begrenzt ist, die sich links und rechts des liegenden Patienten im aufgelegten Zustand der Schale (1) zu beiden Seiten seines Halses erstrecken, wobei die Ausleger (6a, 6b) in einem Bereich zwischen der Aufnahmeaussparung (4) und dem vom Patienten abgewandten Rand der Schale (1) eine Ablagefläche bilden, und wobei der vom Schalenboden (2) und dem umlaufenden Rand (3) gebildete muldenartige Ablagebereich jeweils an den beiden Auslegern (6a, 6b) in dem im aufgelegten Zustand der Schale (1) den Schultern des Patienten benachbarten Bereich seine größte Tiefe aufweist,
**dadurch gekennzeichnet,**
**dass** die Auffangschale (1) derart geformt ist, dass die die Aufnahmeaussparung (4) begrenzenden Ausleger (6a, 6b) im auf dem liegenden Patienten aufgelegten Zustand der Schale (1) den Hals des auf dem Rücken liegenden Patienten (P) mit geringem Abstand zum Patienten beidseitig im Bereich unterhalb der Ohren in einer ersten Ebene (E1) umgibt,
**dass** der an die Aufnahmeaussparung (4) anschließende Zentralbereich (5) der Auffangschale (1) sich im aufgelegten Zustand der Schale (1) zumindest über einen Teil der Brust des Patienten in einer oberhalb der ersten Ebene (E1) parallel zu dieser verlaufenden zweiten Ebene (E2) unterhalb des Kinns des auf dem Rücken liegenden Patienten (P) erstreckt,
und **dass** der vom Schalenboden (2) und dem umlaufenden Rand (3) gebildete Ablagebereich in dem im aufgelegten Zustand der Schale (1) auf der Brust des Patienten aufliegenden Bereich eine gegenüber dem umgebenden Schalenboden (2) tiefere Mulde (8) bildet.

2. Auffangschale nach Anspruch 1, **dadurch gekennzeichnet, dass** die beiden Ausleger (6a, 6b), die Aufnahmeaussparung (4) und der Zentralbereich (5) der Auffangschale (1) in einer Draufsicht auf die Schalenfläche die Form eines stilisierten Backenzahnes aufweisen, dessen Zahnwurzeln durch die beiden Ausleger (6a, 6b) gebildet wird.

3. Auffangschale nach einem der vorhergehenden Ansprüche, **dadurch gekennzeichnet, dass** die Aufnahmeaussparung (4) an ihrem während der Behandlung dem Patienten zuwandten Rand eine Polsterung (7) aufweist.

4. Auffangschale nach Anspruch 3, **dadurch gekennzeichnet, dass** die Polsterung (7) nur den Randbereich der Aufnahmeaussparung (4) umfasst, welcher während der Behandlung dem Hals des Patienten nahekommt.

5. Auffangschale nach einem der Ansprüche 3 oder 4, **dadurch gekennzeichnet, dass** die Polsterung (7) so gestaltet ist, dass sie während der Behandlung das Kinn des Patienten unterstützt und/oder anhebt.

6. Auffangschale nach einem der Ansprüche 3 bis 5, **dadurch gekennzeichnet, dass** die Polsterung (7) aus Weichmaterial, insbesondere Zellstoff oder Weich-Kunststoff aufgebaut ist.

7. Auffangschale nach einem der Ansprüche 3 bis 6, **dadurch gekennzeichnet, dass** das Material der Polsterung (7) so gewählt ist, dass die Polsterung (7) während der Behandlung des Patienten den Raum zwischen Patient und Auffangschale (1) flüssigkeitsdicht abdichtet.

8. Auffangschale nach einem der vorhergehenden Ansprüche, **dadurch gekennzeichnet, dass** am umlaufenden Rand (3) im Bereich der Aufnahmeaussparung (4) patientenseitig zumindest abschnittsweise Klettband angeordnet ist.

9. Auffangschale nach einem der vorhergehenden Ansprüche, **dadurch gekennzeichnet, dass** der umlaufende Rand (3) gebördelt ist.

10. Auffangschale nach Anspruch 9, **dadurch gekennzeichnet, dass** die Bördelung des umlaufenden Randes (3) an ihrem oberen Ende über den Schalenboden (2) überhängend gestaltet ist.

11. Auffangschale nach einem der Ansprüche 9 oder 10, **dadurch gekennzeichnet, dass** in dem im aufgelegten Zustand der Schale (1) auf der Brust des Patienten aufliegenden Bereich keine Bördelung des umlaufenden Randes (3) vorgesehen ist.

12. Auffangschale nach einem der vorhergehenden Ansprüche, **dadurch gekennzeichnet, dass** die Schale (1) zur einmaligen Verwendung und anschließenden Entsorgung vorgesehen ist.

13. Auffangschale nach Anspruch 12, **dadurch gekennzeichnet, dass** die Schale (1) aus einem zumindest über einen Zeitraum von mehr als 30min feuchtigkeitsdichten Material, vorzugsweise aus Presspappe Material besteht.

14. Auffangschale nach einem der Ansprüche 1 bis 12, **dadurch gekennzeichnet, dass** der umlaufende Rand (3) aufblasbar gestaltet ist.

## Claims

1. A collection tray (1) for applications in response to a medical and/or cosmetic treatment of a patient (P), in which in particular medical instruments and aids as well as dental prostheses, if applicable, can be collected at least during the time period of the treatment within the reach of an attending person, in particular a physician, wherein the collection tray (1) comprises a tray bottom (2), which is designed as storage surface and which is surrounded by a circumferential edge (3), wherein the collection tray (1) encompasses an accommodating recess (4), which extends from a partial area of the circumferential edge (3) in the direction of a central area (5) of the tray (1) and which is formed such that it surrounds the neck of a patient lying on the back at a slight distance to the patient on both sides approximately into the area of the ears,
wherein the accommodating recess (4) is defined by two cantilever arms (6a, 6b), which extend to the left and the right of the lying patient on both sides of his neck when the tray (1) is in the applied state, wherein the cantilever arms (6a, 6b) form a storage surface in an area between the accommodating recess (4) and the edge of the tray (1), which faces away from the patient, and wherein the cavity-like storage area, which is formed by the tray bottom (2) and the circumferential edge (3), in each case encompasses its largest depth at the two cantilever arms (6a, 6b) in the area, which is adjacent to the shoulders of the patient when the tray (1) is in the applied state,
**characterized in**
**that** the collection tray (1) is formed such that the cantilever arms (6a, 6b), which define the accommodating recess (4), surround the neck of the patient (P) lying on the back at a slight distance to the patient on both sides in the area below the ears in a first plane (E1) when the tray (1) is in the applied state on the lying patient,
**that**, when the tray (1) is in the applied state, the central area (5) of the collection tray (1), which adjoins the accommodating recess (4), extends at least across a part of the chest of the patient in a second plane (E2), which runs above the first plane (E1) parallel to the latter, below the chin of the patient (P) lying on the back,
and **that** the storage area formed by the tray bottom (2) and the circumferential edge (3) forms a cavity (8), which is deeper as compared to the surrounding tray bottom (2), in the area, which bears on the chest of the patient when the tray (1) is in the applied state.

2. The collection tray according to claim 1, **characterized in that**, in a top view onto the tray surface, the two cantilever arms (6a, 6b), the accommodation recess (4) and the central area (5) of the collection tray (1) encompass the shape of a stylized molar, the tooth roots of which are formed by the two cantilever arms (6a, 6b).

3. The collection tray according to any one of the preceding claims, **characterized in that** the accommodation recess (4) encompasses a cushioning (7) on its edge, which faces the patient during treatment.

4. The collection tray according to claim 3, **characterized in that** the cushioning (7) comprises only the edge area of the accommodation recess (4), which comes close to the neck of the patient during treatment.

5. The collection tray according to any one of claims 3 or 4, **characterized in that** the cushioning (7) is designed such that it supports and/or lifts the chin of the patient during treatment.

6. The collection tray according to any one of claims 3 to 5, **characterized in that** the cushioning is made of soft material, in particular pulp or soft plastic.

7. The collection tray according to any one of claims 3 to 6, **characterized in that** the material of the cushioning (7) is chosen such that the cushioning (7) seals the space between patient and collection tray (1) in a liquid-tight manner during treatment of the patient.

8. The collection tray according to any one of the preceding claims, **characterized in that** a hook and loop fastener is arranged at least in sections on the circumferential edge (3) in the area of the accommodation recess (4) on the side of the patient.

9. The collection tray according to any one of the preceding claims, **characterized in that** the circumferential edge (3) is flanged.

10. The collection tray according to claim 9, **characterized in that**, at its upper end, the flanging of the circumferential edge (3) is designed so as to project beyond the tray bottom (2).

11. The collection tray according to any one of claims 9 or 10, **characterized in that** no flanging of the circumferential edge (3) is provided in the area, which bears on the chest of the patient when the tray (1) is in the applied state.

12. The collection tray according to any one of the preceding claims, **characterized in that** the tray (1) is provided for the one-time use and the subsequent disposal.

13. The collection tray according to claim 12, **characterized in that** the tray (1) consists of a material, which is moisture-tight at least for a time period of more than 30 minutes, preferably of pressboard material.

14. The collection tray according to any one of claims 1 to 12, **characterized in that** the circumferential edge (3) is designed so as to be inflatable.

## Revendications

1. Cuvette collectrice (1) pour des applications lors d'un traitement médical et/ou cosmétique d'un patient (P), dans laquelle notamment des instruments et moyens auxiliaires médicales ainsi qu'éventuellement également une prothèse dentaire peuvent être attrapés au moins pendant la période du traitement à portée d'une personne traitante, notamment d'un médecin, dans laquelle la cuvette collectrice (1) comprend un fond de cuvette (2) configuré en tant que surface de dépôt qui est entouré d'un bord périphérique (3), dans laquelle la cuvette collectrice (1) présente un évidement de réception (4) qui s'étend d'une région partielle du bord périphérique (3) en direction d'une région centrale (5) de la cuvette (1) et est moulé de telle sorte qu'il entoure le cou d'un patient allongé sur le dos à faible distance du patient des deux côtés environ jusque dans la région des oreilles,
dans laquelle l'évidement de réception (4) est limité par deux bras (6a, 6b) qui s'étendent à gauche et à droite du patient allongé à l'état posé de la cuvette (1) des deux côtés de son cou, dans laquelle les bras (6a, 6b) forment dans une région entre l'évidement de réception (4) et le bord de la cuvette (1) détourné du patient une surface de dépôt, et dans laquelle la région de dépôt en forme d'auge, formée par le fond de cuvette (2) et le bord périphérique (3) présente à chaque fois au niveau des deux bras (6a, 6b) dans la région voisine des épaules du patient dans l'état posé de la cuvette (1) sa profondeur la plus grande,
**caractérisée en ce**
**que** la cuvette collectrice (1) est moulée de telle sorte que les bras (6a, 6b) limitant l'évidement de réception (4) entourent à l'état posé sur le patient allongé de la cuvette (1) le cou du patient (P) allongé sur le dos à faible distance du patient des deux côtés dans la région en dessous des oreilles dans un premier plan (E1),
**que** la région centrale (5) de la cuvette collectrice (1) adjacente à l'évidement de réception (4) s'étend à l'état posé de la cuvette (1) au moins sur une partie de la poitrine du patient dans un second plan (E2) s'étendant au-dessus du premier plan (E1) parallèlement à celui-ci en dessous du menton du patient (P) allongé sur le dos,
et **que** la région de dépôt formée par le fond de cuvette (2) et le bord périphérique (3) forme dans la région reposant sur la poitrine du patient à l'état posé de la cuvette (1) une auge (8) plus profonde par rapport au fond de cuvette environnant (2).

2. Cuvette collectrice selon la revendication 1, **caractérisée en ce que** les deux bras (6a, 6b), l'évidement de réception (4) et la région centrale (5) de la cuvette collectrice (1) présentent dans une vue de dessus sur la surface de cuvette la forme d'une molaire stylisée dont les racines sont formées par les deux bras (6a, 6b).

3. Cuvette collectrice selon l'une quelconque des revendications précédentes, **caractérisée en ce que** l'évidement de réception (4) présente sur son bord tourné vers le patient pendant le traitement un rembourrage (7).

4. Cuvette collectrice selon la revendication 3, **caractérisée en ce que** le rembourrage (7) ne comprend que la région de bord de l'évidement de réception (4) qui s'approche du cou du patient pendant le traitement.

5. Cuvette collectrice selon l'une quelconque des revendications 3 ou 4, **caractérisée en ce que** le rembourrage (7) est configuré de sorte qu'il soutient et/ou soulève le menton du patient pendant le traitement.

6. Cuvette collectrice selon l'une quelconque des revendications 3 à 5, **caractérisée en ce que** le rembourrage (7) est construit en matériau mou, notamment cellulose ou plastique mou.

7. Cuvette collectrice selon l'une quelconque des revendications 3 à 6, **caractérisée en ce que** le matériau du rembourrage (7) est choisi de sorte que le rembourrage (7) étanchéifie de manière étanche au liquide l'espace entre le patient et la cuvette collectrice (1) pendant le traitement du patient.

8. Cuvette collectrice selon l'une quelconque des revendications précédentes, **caractérisée en ce que** du ruban autoagrippant est disposé du côté du patient au moins par section sur le bord périphérique (3) dans la région de l'évidement de réception (4).

9. Cuvette collectrice selon l'une quelconque des revendications précédentes, **caractérisée en ce que** le bord périphérique (3) est replié.

10. Cuvette collectrice selon la revendication 9, **caractérisée en ce que** le repli du bord périphérique (3) est configuré en porte-à-faux à son extrémité supérieure au-dessus du fond de cuvette (2).

11. Cuvette collectrice selon l'une quelconque des revendications 9 ou 10, **caractérisée en ce qu'**aucun repli du bord périphérique (3) n'est prévu dans la région reposant sur la poitrine du patient à l'état posé de la cuvette (1).

12. Cuvette collectrice selon l'une quelconque des revendications précédentes, **caractérisée en ce que** la cuvette (1) est prévue pour un usage unique et une élimination subséquente.

13. Cuvette collectrice selon la revendication 12, **caractérisée en ce que** la cuvette (1) se compose d'un matériau étanche à l'humidité au moins sur une période de plus de 30 min, de préférence de matériau en carton comprimé.

14. Cuvette collectrice selon l'une quelconque des revendications 1 à 12, **caractérisée en ce que** le bord périphérique (3) est configuré de manière à pouvoir être gonflé.
